# EUROPEAN PATENT SPECIFICATION

(11) **EP 1 757 293 B1**
(45) Date of publication and mention of the grant of the patent: **10.10.2012**
(21) Application number: 05739251.6
(22) Date of filing: 11.05.2005
(51) Int. Cl.: A61K 31/519, A61J 3/02, A61K 9/10, A61K 9/14, A61K 9/16, A61K 47/02, A61K 47/20, A61K 47/22, A61P 3/00, A61K 9/00

(54) **BH4-RESPONSIVE HYPERPHENYLALANINEMIA REMEDIES**
BH4-RESPONSIVE HYPERPHENYLALANINÄMIE-MITTEL
REMEDES CONTRE L'HYPERPHENYLALANINEMIE SENSIBLE A LA BH4

(30) Priority: 11.05.2004 JP 2004141615
(43) Date of publication of application: 28.02.2007
(73) Proprietor: Daiichi Sankyo Company, Limited, Chuo-ku Tokyo (JP)
(72) Inventor: SUGITA, Osamu, Tokyo 1740056 (JP); MATSUMOTO, Masako, Gunma 3730806 (JP); TAKAI, Tomokazu, Tatebayashi-shi Gunma 3740075 (JP)
(74) Representative: Albrecht, Thomas
(86) International application number: PCT/JP2005/008613
(87) International publication number: WO 2005/107759

(56) References cited:
- WO-A1-99/16470
- JP-A- 2003 277 265
- 'Iryoyaku Nippon Iyakuhinshu.', 2000, NIPPON IYAKU JOHO CENTER. pages 735 - 736, XP002994295
- 'Biopten Karyu 2.5%.', [Online] 2003, XP002994296 Retrieved from the Internet: <URL:http://www.maruho.co.jp/medical/pdf/te /bpt_te.pdf> [retrieved on 2005-08-11]
- 'Japan Pharmaceutical Excipients Council, Iyaku Tenkabutsu Jiten Tsuiho.' 1995, pages 151 - 152, XP002994297

## Description

### TECHNICAL FIELD

The present invention relates to therapeutic agents for tetrahydrobiopterin-responsive hyperphenylalaninemia. In particular, the present invention relates to a therapeutic agent for tetrahydrobiopterin-responsive hyperphenylalaninemia provided in the form of granules, fine granules, or dry syrups containing sapropterin hydrochloride as an active ingredient.

### BACKGROUND ART

Hyperphenylalaninemia is a congenital metabolic disorder in which the plasma level of phenylalanine (Phe) remains elevated. The disease is divided into two types based on what enzyme is deficient: One is phenylketonurea (PKU), also known as PAH deficiency, caused by a defective phenylalanine hydroxylase (PAH) and the other is tetrahydrobiopterin deficiency caused by a disrupted biosynthesis of tetrahydrobiopterin (BH4), a coenzyme of PAH.

Either disease is inherited as an autosomal recessive trait and is characterized by the body's inability to metabolize phenylalanine to tyrosine and the resulting elevation in the plasma Phe level. Hyperphenylalaninemia severely impairs functions of the central nervous system. No apparent symptoms are seen in newborns but psychomotor retardation gradually sets in five to six months after birth, often manifesting spasms and other severe symptoms. When left untreated, the disease leads to severe mental defects. Other symptoms include abnormal EEG and melanin deficiencies, such as red hair and white skin. Thus, neonates are screened for hyperphenylalaninemia during the neonatal period (three to five days after birth) before the onset of symptoms. The governments of US, European countries since 70's, and Japan since 1977 have taken this approach.

The newborn screening is a test designed to measure the blood Phe level. Feeding with Phe-containing breast or artificial milk will be stopped for the newborns diagnosed by the screening test as having phenylketonuria. The babies then follow a special diet regimen by receiving special Phe-free milk and continue to remain on a low-Phe diet as they grow. This diet therapy is intended to keep the Phe intake low and maintain a constant blood Phe level.

Literally all protein-containing food materials contain phenylalanine as part of their proteins. Thus, the low-Phe diet therapy generally requires avoiding protein food and supplying by the "therapeutic milk" the other essential amino acids that are also depleted from food. Such diet therapies not only cost patients and their family members much effort and expense, but they may sometimes be painful to patients who follow the strict diet regimen.

It was previously considered that the patients would need to receive the low-Phe diet until they reach age 15, by which time the brain has been fully developed. However, the follow-up studies of patients have revealed that the patients need to stay on a low-Phe diet throughout their entire lives. It has been reported that when pregnant women with phenylketonuria fail to control their blood Phe levels prior to or during the pregnancy and as a result have a high blood Phe level, the newborns will have a high risk of mental retardation or microencephaly.

In addition to diet therapies, a naturally occurring type of tetrahydrobiopterin (commonly known as "sapropterin hydrochloride" or "sapropterin" in INN) has been developed as a treatment for atypical hyperphenylalaninemia, the BH4 deficiency caused by a deficiency in dihydrobiopterin synthetase or dihydropteridine reductase. To compensate for tyrosine depletion and maintain the blood Phe level, sapropterin hydrochloride is generally administered alone or in combination with a Levodopa preparation or 5-hydroxytryptophan, each a precursor of a tyrosine-derived neurotransmitter. The drug, however, may not provide sufficient effect and is often used with the diet therapy.
BH4 cannot decrease the blood Phe level when administered to patients with phenylalanine hydroxylase (PAH) deficiency or phenylketonuria (PKU) caused by PAH deficiency. Thus, the low-Phe diet therapy has been considered the only effective treatment for these diseases.

Since 1999, cases have been reported in the differential diagnostics of BH4 deficiency in which the blood Phe level decreases during the BH4 tolerance test in response to administration of BH4 despite the fact that the results of the blood or urine pteridine analysis or the red blood cell DHPR assay test suggest the presence of PAH deficiency.
These cases have been identified as a new disease and are termed as BH4-responsive hyperphenylalaninemia or BH4-responsive PAH deficiency. Since administration of sapropterin hydrochloride, or BH4, alone has been proven effective in the treatment of BH4-responsive hyperphenylalaninemia, for which the only available treatment has been a diet therapy, development of effective treatments for the disease, in particular, development of effective sapropterin hydrochloride preparations is an urgent task.

Treatment of BH4-responsive hyperphenylalaninemia must be started immediately once a newborn is diagnosed with the disease in the newborn screening. For this reason, sapropterin hydrochloride should be provided in a dosage form suitable for use in newborns.
In fact, the dosage form of sapropterin hydrochloride should be suitable for use in patients of all ages since patients need to remain on the drug throughout their entire lives.

Since patients may range over all ages from newborns a few days after birth, to adults and to seniors, the preparation of sapropterin hydrochloride must contain the drug in different doses based on the body weight (or age) of the patients, or it must be provided in a dosage form that can readily provide the drug in different doses based on the body weight (or age) of the patients. To facilitate formulation, the dosage form preferably allows simple determination of the dose.

For example, when sapropterin hydrochloride is used to treat atypical hyperphenylalaninemia caused by a deficiency in dihydrobiopterin synthetase or dihydropteridine reductase, the drug is administered at a dose of 2 to 5 mg/kg/day. This corresponds to 6 to 15 mg/day for a 3 kg newborn and 120 to 300 mg/day for a 60 kg adult. Thus, the dose range of sapropterin hydrochloride that has to be delivered by a given preparation is considerably wider than that of other drugs.

Pharmaceutical products that are frequently administered to patients of different age groups, ranging from infants, to children and to adults are often prepared in two different preparations: for child and for adult. A congenital metabolic disorder, hyperphenylalaninemia is an extremely rare disease, however. The total number of cases found by the newborn mass-screening tests conducted in Japan between 1977 and 1988 was 405, of which 247 were phenylketonuria and 17 were BH4 deficiency. This is equivalent to only one in every 600,000 babies.
While the drug is essential to the patients, it is desirable, from the viewpoint of medical personnel, that the drug be formulated in a single preparation that can be given to patients of all ages due to the production scale and storage.
However, the low storage stability and irritancy of sapropterin hydrochloride require development of more effective preparations than those used today.

Patent Document 1: Japanese Patent No. 2761104
Patent Document 2: Japanese Patent Laid-Open Publication No. Sho 61-277618
Patent Document 3: Japanese Patent Laid-Open Publication No. Sho 63-267781
Patent Document 4: Japanese Patent Laid-Open Publication No. Hei 10-338637
Non-Patent Document 1: S. Kure et al. "Tetrahydrobiopterin-responsive phenylalanine hydroxylase deficiency" Journal of Pediatrics, vol. 135, No. 3, 1999, pp. 375-378
Non-Patent Document 2: R. Cerone et al. "Long-term follow-up of a patient with mild tetrahydrobiopterin-responsive phenylketonuria" Molecular Genetics and Metabolism 81 (2004), pp. 137-139
Non-Patent Document 3: "Foundation and activity of Special Committee for Selecting Therapeutic Standards for the Treatment of Tetrahydrobiopterin (BH4)-Responsive Hyperphenylalaninemia" Tokusyu Milk Jijo, No. 30, Nov (2000), pp. 77-79
Non-Patent Document 4: "Research Report 1 by Special Committee for Selecting Therapeutic Standards for the Treatment of Tetrahydrobiopterin (BH4)-Responsive Hyperphenylalaninemia" Tokusyu Milk Jijo, No. 37, Nov (2001), pp. 71-73
Non-Patent Document 5: "Research Report 2 by Special Committee for Selecting Therapeutic Standards for the Treatment of Tetrahydrobiopterin (BH4)-Responsive Hyperphenylalaninemia" Tokusyu Milk Jijo, No. 38, Nov (2002), pp. 44-59

### DISCLOSURE OF THE INVENTION

### PROBLEMS TO BE SOLVED BY THE INVENTION

In view of the above-described current state of the art, it is an object of the present invention to provide an effective sapropterin hydrochloride preparation that is highly stable during storage and can administer sapropterin hydrochloride, an effective therapeutic agent for the treatment of tetrahydrobiopterin-responsive hyperphenylalaninemia, to patients of a wide range of ages, ranging from infants to adults, in a single preparation.

### MEANS FOR SOLVING THE PROBLEMS

Essential aspects of the present invention comprise the following:
(1) A pharmaceutical preparation for the treatment of BH4-responsive hyperphenylalaninemia provided in the form of granules, fine granules or dry syrup, comprising:
   2.5 to 20 wt% of sapropterin hydrochloride as an active ingredient; and
   magnesium aluminometasilicate or dibasic calcium phosphate dihydrate as a flavoring agent;
   wherein the preparation is suitable for use in patients of all ages, ranging from infants to adults.
(2) The pharmaceutical preparation according to (1), further containing a coloring agent which is stable against acid and oxidation.
(3) The pharmaceutical preparation according to (1) or (2), wherein the moisture content of the preparation is 0.9% or less.
(4) The pharmaceutical preparation according to any of (1) to (3), further containing ascorbic acid or L-cysteine hydrochloride as a stabilizer.
(5) The pharmaceutical preparation according to any of (1) to (4), containing 5 to 10 wt% of sapropterin hydrochloride as an active ingredient.
(6) The pharmaceutical preparation according to any of (1) to (5), wherein during the preparation process, the flavoring agent is mixed and kneaded with sapropterin hydrochloride in the presence of water.
(7) The pharmaceutical preparation according to (2), wherein the coloring agent is riboflavin.
(8) The pharmaceutical preparation according to any of (1) to (7), wherein the moisture content of the preparation (loss on drying) is controlled by drying formed granules in a fluidized bed dryer.
(9) The pharmaceutical preparation according to any of (1) to (8) wherein the moisture content of the preparation (loss on drying) is controlled by drying formed granules in a fluidized bed dryer for 15 minutes or more.
(10) The pharmaceutical preparation according to any of (1) to (9), wherein the granule, fine granule, or dry syrup is contained in a package that blocks external moisture.
(11) The pharmaceutical preparation according to any of (1) to (10), wherein the granule, fine granule, or dry syrup is contained in a package that blocks external moisture using a moisture-proof packaging material.
(12) The pharmaceutical preparation according to (11), wherein the moisture-proof packaging material has a moisture permeability of less than 30 g/m²/day.
(13) The pharmaceutical preparation according to (1), further containing riboflavin as a coloring agent, wherein the preparation has a moisture content of 0.9% or less and the preparation comprises 5 to 10 wt% of sapropterin hydrochloride.

### ADVANTAGES OF THE INVENTION

The preparation of the present invention can be used to administer sapropterin hydrochloride, an effective treatment for tetrahydrobiopterin-responsive hyperphenylalaninemia, to patients of a wide range of ages, ranging from infants to adults, in a single preparation. Not only is the preparation highly effective, but it also has good ingestibility and is stable during storage.
Thus, the preparation can be applicable to patients of all ages, ranging from infants, who are incapable of swallowing, to elderlies, who are having difficulty swallowing, so as to deliver the drug in a wide range of doses required by the different age groups.

### BEST MODE FOR CARRYING OUT THE INVENTION

The present invention will now be described in specific detail with reference to examples and test examples in which different preparations are formulated.

In the course of our effort to formulate a preparation containing sapropterin hydrochloride, the present inventors have drawn attention to the fact that sapropterin hydrochloride can be administered to patients of a wide range of ages, ranging from newborns to adults, in a single preparation when it is formulated in such dosage forms as powders (*e.g.*, granules and fine granules) and dry syrups.
Unlike dosage forms such as tablets and capsules, which are unsuitable for administration to infants, who are incapable of swallowing, and elderlies, who have difficulty swallowing, powders (*e.g.*, granules and fine granules) and dry syrups are readily ingestible by these patients. In addition, these dosage forms may be formed into solutions when necessary.

Furthermore, powders (*e.g.*, granules and fine granules) and dry syrups are suitable for delivering the drug in a wide range of doses. If tablets, for example, are used to cover such a wide range of doses, such tablets must be formulated such that a single tablet contains a single dose of the drug intended for an infant. Since one such tablet gives the minimum dose unit, adults may need to take up to several tens of these tablets at a time to meet their required doses. This is impractical.
The preparations provided in the form of powders (*e.g.*, granules and fine granules) and dry syrups are also preferred since the speed at which the drug passes the gastrointestinal tract does not significantly deviate when it is administered in these dosage forms.

When the preparation provided in the form of powders (*e.g.*, granules and fine granules) and dry syrups is intended for use in infants and small children, it is preferred that the preparation is formulated in as small a volume as possible since these patients have difficulty orally ingesting large volumes. Taking this aspect into account, the preparation contains sapropterin hydrochloride, the active ingredient, in an amount of 2.5% to 20% and more preferably in an amount of 5% to 10%. The preparation most preferably contains the drug in an amount of 10% since such preparation facilitates the prescription of the drug in different doses and helps reduce the risk of miss-prescription.

In formulating sapropterin hydrochloride into a preparation in the form of powders (*e.g.*, granules and fine granules) and dry syrups, the following points need to be considered:
(1) The unpleasant sour or acrid taste of the preparation that results from the hydrochloride nature of sapropterin hydrochloride must be reduced.
(2) The preparation must be stable.
(3) Sapropterin hydrochloride, the active ingredient, is in itself likely to decompose in the presence of moisture. Thus, the stability of sapropterin hydrochloride must be ensured during the process to form the drug into a preparation or in the event that the drug comes into contact with destabilizing materials used in the process.

We thus formulated granule preparations containing 2.5%, 5% and 10% of sapropterin hydrochloride and a fine granule preparation containing 10% of sapropterin hydrochloride.

### Example 1: Preparations containing different amounts of active ingredient, and their ingestibility.

Different granule preparations containing 2.5%, 5% and 10% of sapropterin hydrochloride, as well as a fine granule preparation containing 10% of sapropterin hydrochloride, were prepared in the manner as described below. Each of the preparations had a formulation (Amounts of components given in mg) as shown in Table 1 below. Each preparation was evaluated for ingestibility.

Each granule preparation was prepared in the following manner: Sapropterin hydrochloride, D-mannitol and low-substituted hydroxypropylcellulose were mixed together. Meanwhile, riboflavin was dispersed in an aqueous solution containing povidone, ascorbic acid, and L-cysteine hydrochloride to make a binder solution. The binder solution was added to the sapropterin hydrochloride mixture. Water was then added and the mixture was kneaded. The kneaded product was extruded into granules, which in turn were dried and sized to obtain a desired granule preparation.
The fine granule preparation was prepared in the following manner: Sapropterin hydrochloride and D-mannitol were mixed together. Meanwhile, riboflavin was dispersed in an aqueous solution containing hydroxypropylcellulose to make a binder solution. The sapropterin hydrochloride mixture was granulated in a fluidized bed granulation process while being sprayed with the binder solution. The granules were dried and sized to obtain a desired preparation.

**(Table 1)**

| Preparation Examples | | 1 | 2 | 3a | 4 |
|---|---|---|---|---|---|
| Active ingredient or additives | | 2.5% Granule | 5% Granule | 10% Granule | 10% Fine granule |
| Active ingredient | Sapropterin hydrochloride | 25.0 | 50.0 | 100.0 | 100.0 |
| Excipient | D-mannitol | q.s. | q.s. | q.s. | q.s. |
| Disintegrating agent | Low-substituted hydroxypropylcellulose (L-HPC) | 50.0 | 50.0 | 50.0 | - |
| Binder | Povidone | 12.0 | 12.0 | 12.0 | - |
| Binder | Hydroxypropylcellulose (HPC) | - | - | - | 25.0 |
| Stabilizer | Ascorbic acid | 5.0 | 5.0 | 5.0 | - |
| Stabilizer | L-cysteine hydrochloride | 2.5 | 2.5 | 2.5 | - |
| Coloring agent | Yellow No. 5 aluminum lake for food use | Trace amount | - | - | - |
| Coloring agent | Riboflavin | - | Trace amount | Trace amount | Trace amount |
| Flavoring agent | Flavor material | Trace amount | - | - | - |
| Total | | 1000 | 1000 | 1000 | 1000 |

### Evaluation by ingestibility 1

Each of the preparations obtained above was evaluated for ingestibility, as follows:
Subjects: Three adults in the twenties to forties.
Test Procedure: Subjects were asked to ingest 0.5 g of each preparation and keep the preparation at their pharynx for about 5 seconds. They were then asked to rate the acridness they felt on a scale of 1 to 5 with 1 being "most acrid" and 5 being "non-acrid."
The results were summarized in Table 2 below.

**(Table 2)**

| Preparation Examples | 1 | 2 | 3a | 4 |
|---|---|---|---|---|
| Preparations | 2.5% Granule | 5% Granule | 10% Granule | 10% Fine granule |
| Ingestibility (acridity) | 4.0 | 3.6 | 1.7 | 2.0 |

As can be seen from the results of Table 2, the 2.5% granule preparation of Preparation Example 1 was rated as slightly acrid though the acridness was not so strong as to make its ingestion difficult. The 5% granule preparation of Preparation Example 2 was rated as moderately acrid and its ingestibility was relatively low. The 10% granule preparation of Preparation Example 3a and the 10% fine granule preparation of Preparation Example 4 were each rated as strongly sour and acrid, indicating very low ingestibility.

We thus conducted studies to find approaches to reduce the sour or acrid taste of the preparation.
When unpleasant tastes, such as bitter taste, occur in granules or dosage forms due to the presence of active ingredients, a coating is generally applied to conceal such tastes. Not only does this approach complicate the production process, but the coating agent may also affect the oral absorption, uniformity and other properties of the preparation.

Thus, we further conducted studies to establish an approach to improve the ingestibility of the preparation without affecting the physical properties of the preparation, such as disintegrating property, and with simple preparation process.

To reduce the sour and acrid taste of the preparation, we used sucrose, fructose, sodium saccharide and other strong sweeteners as a substitute for D-mannitol to see if strong sweeteners could weaken the perceived sour taste of the preparation. It turned out that the sour taste was reduced and the ingestibility of the preparation was improved to some extent when sucrose, fructose, or sodium saccharide was added. Thus, we prepared preparations using these three sweeteners and studied their stability over time, only to find that in each case the appearance of these preparations was significantly affected over time, as described in Example 2 below.

### Example 2

Sapropterin hydrochloride was mixed at a 1:5 ratio with each of the three sweeteners described above. 2 g of the mixture was placed in a glass vial and the vial was left uncapped at 40°C, 75% RH, to evaluate the stability of the preparation and sapropterin hydrochloride. Unlike the sweetener-free preparation, which maintained the same appearance and kept the comparable content of active ingredient after an 8-week storage period, all of the sweetener-added preparations changed their color after 2 weeks of storage. The preparations using sucrose and fructose even underwent deliquescence. These results are shown in Table 3. The remaining sapropterin hydrochloride was quantified by liquid chromatography that can differentiate sapropterin hydrochloride from its analogues. The area of the peak for sapropterin hydrochloride was determined and was used to calculate the remaining sapropterin hydrochloride (The same procedure was followed in other examples).

**(Table 3)**

| Sweeteners | Initial appearance | Storage period | Appearance after storage | % amount of remaining sapropterin hydrochloride after storage |
|---|---|---|---|---|
| None | Yellowish white | 8 weeks | Yellowish white | 98.7 |
| Sucrose | Yellowish white | 2 weeks | Very dark yellowish red (Deliquescence) | Not measured |
| Fructose | Yellowish white | 2 weeks | Very dark yellowish red (Deliquescence) | Not measured |
| Sodium saccharide | Yellowish white | 2 weeks | Dark reddish yellow | Not measured |

We therefore attempted to decrease the sensitivity of the oral cavity to the taste and acridness of the drug, rather than conceal the sour taste by sweeteners, and added as a flavoring agent a substance that can mask the taste of the hydrochloride and decrease the direct sensitivity of the oral cavity. Of many pharmaceutically acceptable substances that we have examined the possibility of using in the preparation of the present invention, antacids, compounds that can decrease the acidity of hydrochloride, seemed to be effective and we first studied them.

Examples of the flavoring agent substance include magnesium aluminometasilicate, dibasic calcium phosphate dihydrate, calcium carbonate, calcium acetate, sodium bicarbonate, magnesium carbonate, disodium hydrogen phosphate dodecahydrate and sodium polyphosphate. Of these, calcium acetate was excluded because of its limited dose (maximum daily dose = 9.2 mg). We thus formulated preparations containing the other flavoring agents and studied their ingestibility.

### Example 3: Addition of flavoring agents and ingestibility

Different granule preparations, each containing 10% sapropterin hydrochloride, were prepared according to the formulas shown in Table 4 below (Amounts of components given in mg). These preparations were evaluated for ingestibility. Each preparation was prepared in the same manner as described in Example 1.
Since 2.5% granule preparations were rated in Example 1 as causing no difficulty upon ingestion, we assumed that the pH range obtained when 2.5% granules were dissolved in an aqueous solution was the optimum pH range and determined the amount of each flavoring agent so that the pH would be maintained within this range.

**(Table 4)**

| Preparation Examples | 5a | 5b | 6a | 7 | 8 | 9 | 10 | 11 |
|---|---|---|---|---|---|---|---|---|
| Sapropterin hydrochloride | 100.0 | 100.0 | 100.0 | 100.0 | 100.0 | 100.0 | 100.0 | 100.0 |
| D-mannitol | q.s. | q.s. | q.s. | q.s. | q.s. | q.s. | q.s. | q.s. |
| L-HPC | 50.0 | 50.0 | 50.0 | 50.0 | 50.0 | 50.0 | 50.0 | 50.0 |
| Magnesium aluminometasilicate | 5.0 | 10.0 | - | - | - | - | - | - |
| Dibasic calcium phosphate dihydrate | - | - | 30.0 - 32.5 | - | - | - | - | - |
| Calcium carbonate | - | - | - | 10.0 - 11.0 | - | - | - | - |
| Sodium bicarbonate | - | - | - | - | 20.0 - 22.5 | - | - | - |
| Magnesium carbonate | - | - | - | - | - | 12.5 | - | - |
| Disodium hydrogen phosphate dodecahydrate | - | - | - | - | - | - | 80.0 | - |
| Sodium polyphosphate | - | - | - | - | - | - | - | 30.0 |
| Povidone | 12.0 | 12.0 | 12.0 | 12.0 | 12.0 | 12.0 | 12.0 | 12.0 |
| Ascorbic acid | 5.0 | 5.0 | 5.0 | 5.0 | 5.0 | 5.0 | 5.0 | 5.0 |
| L-cysteine hydrochloride | 2.5 | 2.5 | 2.5 | 2.5 | 2.5 | 2.5 | 2.5 | 2.5 |
| Riboflavin | Trace amount | Trace amount | Trace amount | Trace amount | Trace amount | Trace amount | Trace amount | Trace amount |
| Total | 1000 | 1000 | 1000 | 1000 | 1000 | 1000 | 1000 | 1000 |

### Evaluation by ingestibility 2

The preparations were evaluated for ingestibility. Preparation Example 3a of Example 1 was also evaluated.
Ingestibility of each preparation was evaluated in the following manner.
Subjects: Three adults in the twenties to forties.
Test Procedure: Subjects were asked to ingest and evaluate Preparation Examples 3a, 5a, 5b, 6a, 7, 8, 9, 10, and 11.
Ingestibility of each preparation was evaluated by the taste and acridness, as follows:
(1) Subjects were asked to verbally evaluate the taste.
(2) Subjects were asked to rate the acridness on a scale of 1 to 5 with 1 being "most acrid" and 5 being "non-acrid." Average scores were compared.
The results are shown in Table 5 below.

**(Table 5)**

| Preparation Examples | Flavoring agent | Taste | Acridness |
|---|---|---|---|
| 3a | None | Strong sour taste | 1.7 |
| 5a | Magnesium aluminometasilicate | Sour taste, slight bitter taste | 3.0 |
| 5b | Magnesium aluminometasilicate | Slight sour taste | 3.7 |
| 6a | Dibasic calcium phosphate dihydrate | Slight sour taste | 3.8 |
| 7 | Calcium carbonate | Slight sour taste | 3.7 |
| 8 | Sodium bicarbonate | Slight sour taste | 4.3 |
| 9 | Magnesium carbonate | Slight sour taste, bitter taste | 3.7 |
| 10 | Disodium hydrogen phosphate dodecahydrate | Slight sour taste, salty taste | 3.7 |
| 11 | Sodium polyphosphate | Sour taste, bitter taste | 3.3 |

The results of Table 5 indicate that each of Preparation Examples 5a, 5b, 6a, 7, 8, 9, 10 and 11 was rated as having a decreased acridness as compared to Preparation Example 3a. Preparation Examples 9, 10 and 11 each had a decreased acridness but were each rated as more of less tasting unpleasantly bitter or salty derived from the flavoring agent. In contrast, each of Preparation Examples 5a, 5b, 6a, 7 and 8 was rated as having little unpleasant taste.

Since Preparation Examples 5a, 6a, 7, and 8 were rated as having no bitter or salty taste derived from the flavoring agent with little acridness, we then tested these preparations for stability. Specifically, 2 g of each granule preparation were placed in glass vials with 0.5 g of silica gel. The vials were sealed with a plastic cap and were stored at 60°C for 2, 4, and 8 weeks. The change in the appearance of each preparation and in the content of the active ingredient was measured, as well as the content of degradation products, the loss on drying, and pH of a solution. Preparation Example 3a was used as a control.

It turned out that Preparation Example 7 containing calcium carbonate turned dark yellow after 2 weeks, showing a significant color change from the initial state, whereas Preparation Example 3a to serve as control remained pale yellow after 8 weeks, showing little change from the initial state. In Preparation Example 7, the percent of initial content of active ingredient was decreased to 98.6% after 2 weeks and to 94.9% after 8 weeks.
Preparation Example 8 containing sodium bicarbonate also turned dark yellow after 2 weeks, showing a significant color change from the initial state. The percent of initial content of active ingredient was decreased to 96.6% after 2 weeks and 93.4% after 8 weeks.
Thus, calcium carbonate (Preparation Example 7) and sodium bicarbonate (Preparation Example 8) both caused color change in the granules and resulted in a significant decrease (to 95% or less) in the amount of the active ingredient after 8 weeks. These flavors were therefore determined to be unfavorable.
The results of the stability test (% of remaining active ingredient) are shown in Table 6 below.

**(Table 6)**

| Storage condition | % amount remained | | | |
|---|---|---|---|---|
| | Preparation Example 5a | Preparation Example 6a | Preparation Example 7 | Preparation Example 8 |
| Initial | 100.0 | 100.0 | 100.0 | 100.0 |
| 60° C, 8 weeks | 96.2 | 96.8 | 94.9 | 93.4 |

### Evaluation by ingestibility 3

We thus evaluated Preparation Examples 5a, 6a and 4 for ingestibility.
Ingestibility of each preparation was evaluated in the following manner.
Subjects: Eleven adults in the twenties to fifties.
Test Procedure: Subjects ingested the granule preparations of Preparation Examples 5a and 6a and the fine granule preparation of Preparation Example 4 in a random sequence and in a blind manner (Subjects did not know what was given) and were asked to fill in a questionnaire.
The questionnaire contained evaluation of the following four taste criteria: (1) sour taste; (2) bitter taste; (3) acridity; and (4) remaining taste of acridity. Each criterion was rated on the following scale (in the decreasing order of ingestibility). Total scores were compared for each criterion.

| | |
|---|---|
| No taste | 0 point |
| Slight taste | 1 point |
| Moderate taste | 2 points |
| Strong taste | 3 points |

The results are summarized in Table 7 below.

**(Table 7)**

| Evaluation criteria | Preparation Example 5a | Preparation Example 6a | Preparation Example 4 |
|---|---|---|---|
| Sour taste | 19 | 11.5 | 25.5 |
| Bitter taste | 7 | 8 | 11 |
| Acridity | 3 | 2 | 12 |
| Remaining taste of acridity | 1 | 1.5 | 9 |

The results indicate that Preparations 5a and 6a tasted less sour and had less bitter taste and acridness than Preparation 4, proving the high ingestibility of Preparations 5a and 6a. Thus, magnesium aluminometasilicate and dibasic calcium phosphate dihydrate are used as flavoring agents in the preparation of the present invention.
Next, we studied processes for mixing these flavoring agents.

### Example 4

100 mg of each of the flavoring agents were added to 900 mg of the 10% fine granule of Preparation Example 4, which was shown to have strong acridity in the preceding experiment. The mixtures were compared with flavoring agent-free control (Preparation Example 4) for taste. To 200 mg of each mixture was dissolved or suspended in 1 mL of purified water and the pH of the solution/suspension was measured. The results are shown in Table 8.

**(Table 8)**

| Flavors | Taste | pH |
|---|---|---|
| None (Control) | Sour taste | 1.4 |
| Calcium carbonate | Strong sour taste and acridity though slightly milder than Control. Sweet taste. | 6.1 |
| Disodium hydrogen phosphate dodecahydrate | Strong sour taste and acridity though slightly milder than Control. Salty taste. | 2.3 |
| Sodium polyphosphate | Strong sour taste and acridity though slightly milder than Control. | 5.2 |

The results of Table 8 indicate that the addition of 10% (by weight) calcium carbonate or sodium polyphosphate as a flavoring agent did not significantly improve the ingestibility (or, did not decrease sour taste or acridity) of the preparation when the flavoring agent was physically mixed into the preparation, while the pH of its solution was near neutral. In contrast, when sapropterin hydrochloride was mixed/kneaded with the flavoring agent in the presence of water as shown in Table 5, the presence of about 1% (by weight) of calcium carbonate or about 3% of sodium polyphosphate was sufficient to decrease the sour taste of the preparation to a degree that allows the preparation to be ingested without difficulty. Disodium hydrogen phosphate dodecahydrate decreased the sour taste of the preparation to an acceptable degree when added in an amount of about 8%.

We then studied processes for mixing sapropterin hydrochloride with the other flavoring agents (*i.e*., magnesium aluminometasilicate, dibasic calcium phosphate dihydrate and sodium bicarbonate).

### Example 5

Each flavoring agent was added to 1,000 mg of the 10% granules of Preparation Example 3a, which was shown to have strong acridity in the preceding experiment. The mixtures were compared with flavoring agent-free control (Preparation Example 3a) for taste. The results are shown in Table 9.

**(Table 9)**

| Flavoring agent | Amount of Flavors | Taste |
|---|---|---|
| None (Control) | None | Strong sour taste and acridity |
| Magnesium aluminometasilicate | 10 mg | Strong sour taste and acridity though slightly milder than Control. |
| Dibasic calcium phosphate dihydrate | 30 mg | Strong sour taste and acridity though slightly milder than Control. |
| Sodium bicarbonate | 20 mg | Strong sour taste and acridity though slightly milder than Control. |

As shown in Table 9, the sour taste and acridity of the preparation were not significantly decreased by physically mixing each of the flavoring agents with sapropterin hydrochloride. In comparison, when the flavoring agents were mixed/kneaded with sapropterin hydrochloride in the presence of water, the resulting preparation had a significantly reduced sour taste or acridity. This is believed to be because simply physically mixing sapropterin hydrochloride with the flavoring agent cannot keep the hydrochloride from directly irritating tongue and pharynx upon ingestion of the preparation, whereas the preparation made by mixing/kneading sapropterin hydrochloride with the flavoring agent in the presence of water can decrease the sensitivity of the oral cavity to taste and acridity since sapropterin hydrochloride is uniformly mixed with the flavoring agent in such preparation.

These results indicate that the flavoring agent acts to significantly decrease the sour taste and acridity of the preparation upon ingestion when it is mixed/kneaded with sapropterin hydrochloride in the presence of water. If simply physically mixed with sapropterin hydrochloride, the flavoring agent cannot significantly improve the ingestibility of the preparation. The mixing/kneading process may be part of the common production process of granules, fine granules and dry syrups, or a separate mixing/kneading process may be provided.

When the flavoring agent is magnesium aluminometasilicate, it is preferably used in an amount of 5 to 10 mg in 1000 mg of a preparation containing 10% sapropterin hydrochloride. The flavoring agent cannot provide the desired effect of decreasing acridity when used in amounts of less than 5 mg, whereas it may cause bitter taste of magnesium aluminometasilicate when used in amounts exceeding 10 mg. When the flavoring agent is dibasic calcium phosphate dihydrate, it is preferably used in an amount of 20 to 40 mg in 1,000 mg of a preparation containing 10% sapropterin hydrochloride. The flavoring agent cannot provide the desired effect of decreasing acridity when used in amounts of less than 20 mg. Although dibasic calcium phosphate dihydrate may be used in amounts greater than 40 mg, it does not have to be used in excess amounts as long as the desired effect is achieved. The flavoring agent is preferably used in an amount of about 0.5% to about 10% of a preparation containing 10% sapropterin hydrochloride. It is preferred that the amount of the flavoring agent used is in a range that does not cause unpleasant taste or smell of the flavoring agent.

These observations together explain why flavors selected from magnesium aluminometasilicate and dibasic calcium phosphate dihydrate are used in the preparation of the present invention. However, a concern remains that these flavoring agents may decrease the stability of aqueous solutions of the preparation since sapropterin hydrochloride is most stable under acidic conditions. Although pharmaceutical preparations in the form of granules, fine granules and dry syrups are generally ingested orally as they are, they may be dissolved in water when, for example, they are administered to infants. We thus further examined the stability of aqueous solutions of the preparation.

### Example 6

Different flavoring agent-added or flavoring agent-free preparations were prepared with or without ascorbic acid and L-cysteine hydrochloride to serve as stabilizers. The preparations were suspended in water and the suspensions were compared for stability.
Specifically, different preparations were prepared according to the formulae shown in Table 10 below (Amounts of components given in mg). 1g of each of preparations were suspended in 25 ml water and the suspension was stored at 25°C for 24 hours under 1,000 LUX condition. Each suspension was then analyzed for the pH, the amount of the remaining active ingredient and the amount of analogues of the active ingredient. The results are shown in Table 11.

**(Table 10)**

| Preparation No. | 3a | 3b | 5a | 5c | 6a | 6b |
|---|---|---|---|---|---|---|
| Sapropterin hydrochloride | 100.0 | 100.0 | 100.0 | 100.0 | 100.0 | 100.0 |
| D-mannitol | q.s. | q.s. | q.s. | q.s. | q.s. | q.s. |
| Low-substituted hydroxypropylcellulose | 50.0 | 50.0 | 50.0 | 50.0 | 50.0 | 50.0 |
| Magnesium aluminometasilicate | - | - | 5.0 | 5.0 | - | - |
| Dibasic calcium phosphate dihydrate | - | - | - | 30.0 | 30.0 | 30.0 |
| Povidone | 12.0 | 12.0 | 12.0 | 12.0 | 12.0 | 12.0 |
| Ascorbic acid | 5.0 | - | 5.0 | - | 5.0 | - |
| L-cysteine hydrochloride | 2.5 | - | 2.5 | - | 2.5 | - |
| Riboflavin | Trace amount | Trace amount | Trace amount | Trace amount | Trace amount | Trace amount |
| Total | 1000 | 1000 | 1000 | 1000 | 1000 | 1000 |

**(Table 11)**

| Preparation Examples | Storage period | pH | % amount of remaining active ingredient | % amount of analogues |
|---|---|---|---|---|
| 3a | Initial | 1.93 | 100 | 0.01 |
| | 25°C, 1000Lux, 24 hrs | 1.91 | 100.1 | 0.13 |
| 3b | Initial | 1.95 | 100 | 0.03 |
| | 25°C, 1000Lux, 24 hrs | 1.93 | 98.3 | 1.93 |
| 5a | Initial | 1.99 | 100 | 0.00 |
| | 25°C, 1000Lux, 24 hrs | 2.03 | 100.5 | 0.41 |
| 5c | Initial | 2.00 | 100 | 0.05 |
| | 25°C, 1000Lux, 24 hrs | 2.04 | 96.4 | 3.24 |
| 6a | Initial | 2.34 | 100 | 0.01 |
| | 25°C, 1000Lux, 24 hrs | 2.33 | 101.3 | 0.31 |
| 6b | Initial | 2.37 | 100 | 0.09 |
| | 25°C, 1000Lux, 24 hrs | 2.33 | 95.6 | 3.82 |

The results demonstrate that when suspended in water, the flavoring agent-added preparations had a slightly less stability than the flavoring agent-free preparations. However, addition of ascorbic acid and L-cysteine hydrochloride to serve as stabilizers maintained the stability. Other stabilizers that can be used in the preparation of the present invention include sodium pyrosulfite, thioglycerol, sodium sulfite, sodium bisulfite and sodium edetate. The amount of ascorbic acid used is preferably from 5 to 20 mg in 1000 mg of a preparation containing 10% sapropterin hydrochloride. The amount of L-cysteine hydrochloride used is preferably from 2.5 to 10 mg in 1,000 mg of preparation. Too little stabilizer results in a decreased stability of the preparation suspended in water, whereas too much stabilizer can lead to a decreased stability of the solid preparation. The addition of the stabilizers did not affect the effects of the flavoring agents such as enhancement of preparation ingestibility and stability of preparation and sapropterin hydrochloride.

Sapropterin hydrochloride, the active ingredient of the present invention, changes color from its original white to light reddish brown or yellow when it decomposes to just a small degree. The decomposition accelerates in the presence of moisture. A preparation containing a large amount of the active ingredient tends to undergo significant color change. Thus, our goal was to make preparations that are less susceptible to color change since the color change may make patients anxious, though not affecting the efficacy of the pharmaceutical preparation.
We first tried an approach for concealing the color change. In general, coatings or coloring agents are applied to pharmaceutical preparations to conceal the color change that takes place over time.

Yellow No. 5 aluminum lake for food use is currently added to the existing 2.5% granule preparations used in the treatment of atypical hyperphenylalaninemia to conceal the color change of the preparation resulting from the color change of the active ingredient.
As is demonstrated below, the strong reducing power of sapropterin hydrochloride causes the coloring agent to decompose and thus lose color when the preparations are stored under moist conditions.

The granules of Preparation Example 1 containing 2.5% sapropterin hydrochloride are highly stable when contained in aluminum packages (See, Table 12 below) but lose or change color once packages are opened and the preparation is left under moist conditions (See, Table 13 below).
The cause of this discoloration is believed to be that sapropterin hydrochloride, a strong reducing agent, reduces yellow No. 5 aluminum lake for food use and causes the coloring agent to lose its color.

**(Table 12)**

| Evaluation criteria | Appearance | % amount of remaining active ingredient | Loss on drying (%) | Disintegration (min) |
|---|---|---|---|---|
| Initial | Aromatic pale orange granule | 100 | 0.10 - 0.19 | Within 1 min |
| 42 months at room temperature | No change | 97.7 - 98.6 | 0.12 - 0.27 | Within 1 min |

**(Table 13)**

| Evaluation criteria | Appearence | % amount of remaining active ingredient | Loss on drying (%) | Disintegration (min) |
|---|---|---|---|---|
| Initial | Aromatic pale orange granule | 100 | 0.10 - 0.19 | Within 1 min |
| 40° C/75% RH, 3 months | Yellowish brown granule with unique weak scent | 80.1 - 87.8 | 0.84 - 1.00 | Within 1 min |

To obtain stable sapropterin hydrochloride preparations that are highly stable under any storage conditions and do not undergo color change over time, proper coloring agents must be used that do not change color over time.
Thus, we added different yellow or red coloring agents, colors favored by many patients, to the preparation to find effective coloring agents. The results are shown in Examples below.

### Example 7: Variation by blending sapropterin hydrochloride with different coloring agents

Sapropterin hydrochloride was mixed at a 200:1 ratio with different coloring agents. 0.2 g of each mixture was placed in a glass vial, which was then sealed with a metal cap and was stored at 40°C and 75% RH for 4 weeks. Subsequently, the mixtures were evaluated for stability (*i.e.*, change in appearance, amount of remaining active ingredient).
The results are summarized in Table 14 below.

**(Table 14)**

| Coloring agent | Initial appearance | After 4-week storage (40°C/75% RH) | |
|---|---|---|---|
| | | Appearance change | % amount remained |
| None | White | Yellowish white (color change) | 99.6 |
| Yellow No. 5 aluminum lake for food use | Pale orange | Pale yellow (discoloration) | 100.7 |
| Iron sesquioxide | Dark pink | Reddish brown (color change) | Not measured |
| Yellow iron sesquioxide | Pale yellow | Reddish brown (color change) | Not measured |
| Riboflavin | Pale yellow | Yellow | 100.4 |

As shown by the results, the preparations containing iron sesquioxide and yellow iron sesquioxide significantly changed their color after 1-week storage period. In comparison, the preparations containing yellow No. 5 aluminum lake for food use and riboflavin did not significantly change in appearance after 4-week storage period. In these preparations, the color change observed in the absence of coloring agents was concealed and the amount of the active ingredient was not decreased. It has turned out that riboflavin is particularly favorable since it is less susceptible to acids and does not affect the active ingredient. Iron sesquioxide and yellow iron sesquioxide, each an oxidizing agent, facilitate decomposition of the active ingredient and are therefore not favorable.

Pharmaceutically acceptable coloring agents other than those used above were used to prepare 10% granule preparations of Preparation Example 3a. The preparations were stored in a harsh environment to see if high stability could be achieved as in the case of riboflavin or the like.

### Example 8: Other coloring agents

10% granule preparations were prepared using, as a coloring agent, riboflavin, cochineal extract (carmine), blue No. 1 for food use, yellow No. 4 aluminum lake for food use, red No. 3 aluminum lake for food use and red No. 106 for food use. The preparations were formulated according to the formula of Preparation Example 3a and the coloring agents were used in place of riboflavin. 2g of each preparation were placed in a glass vial, which was then sealed and was stored at 60°C for 8 weeks. Subsequently, the preparations were evaluated for stability (*i.e.,* change in appearance, amount of remaining active ingredient). The preparations were stored in the presence or absence of silica gel (0.5 g).
The results are shown in Tables 15 and 16 below.

**(Table 15)**

| Coloring agent | Initial appearance | After 8-week storage (60°C, with silica gel) | |
|---|---|---|---|
| | | Appearance | % amount remained |
| Riboflavin | Pale yellowish white | Pale yellow | 94.8 |
| Cochineal extract (carmine) | Bright reddish purple | Bright purplish red | 93.7 |
| Blue No. 1 for food use | Brilliant bluish green, and bright green | Dark bluish green | 95.5 |
| Yellow No. 4 aluminum lake for food use | Slightly greenish yellow | Pale yellow | 94.5 |
| Red No. 106 for food use | Bright reddish purple | Bright reddish purple | 96.5 |

**(Table 16)**

| Coloring agent | Initial appearance | After 8-week storage (60°C, without silica gel) | |
|---|---|---|---|
| | | Appearance | % amount remained |
| Riboflavin | Pale yellowish white | Dark reddish yellow | 93.4 |
| Cochineal extract (carmine) | Bright reddish purple | Bright purplish red | 94.5 |
| Blue No. 1 for food use | Brilliant bluish green, and bright green | Dark bluish green | 94.9 |
| Yellow No. 4 aluminum lake for food use | Slightly greenish yellow | Bright yellow | 95.4 |
| Red No. 3 for food use | Pale yellowish red | Slightly reddish yellow | 94.2 |
| Red No. 106 for food use | Bright reddish purple | Dark purplish red | 94.8 |

As can be seen from the results of the tables above, discoloration was not observed and the amount of the active ingredient was not significantly decreased in any of the granule preparations tested after storage in a harsh environment (*i.e*., 8 weeks at 60°C). Thus, each preparation has been proven to be highly stable.
This observation demonstrates that addition of coloring agents, such as riboflavin, cochineal extract (carmine), blue No. 1 for food use, yellow No. 4 aluminum lake for food use, yellow No. 5 aluminum lake for food use, red No. 3 aluminum lake for food use and red No. 106 for food use, to the preparation of the present invention conceals the discoloration or color change of the active ingredient without decreasing the amount of sapropterin hydrochloride. The coloring agent may be any coloring agent that is not susceptible to acid generated by the active ingredient. The coloring agent may be added in any amount that provides a desired color for a given coloring agent: It is preferably used in an amount of 0.1% or less (i.e., 1 mg or less in 1,000 mg preparation). For example, riboflavin is suitably used in an amount of about 0.01 to about 0.05%.

Sapropterin hydrochloride, the active ingredient of the present invention, changes color from its original white to reddish brown or yellow when it decomposes to just a small degree. Sapropterin hydrochloride decomposes and changes color as it absorbs ambient moisture such as moisture present in trace amounts in the preparation and in aluminum packages. Since decomposition of sapropterin hydrochloride accelerates in the presence of moisture, we conducted an experiment to see if it is possible to ensure stability of sapropterin hydrochloride by controlling the moisture content of the preparation and to minimize the decomposition of sapropterin hydrochloride.
Provided in the form of granules, fine granules or dry syrups, the preparation of the present invention often requires addition of water during its production. Thus, the moisture content of the preparation comes not only from its constituents, but also from water added during production of the preparation. This poses a problem.

### Example 9: Effects of moisture content on the stability of preparation

To address the above-described problem, a granule preparation containing 10% sapropterin hydrochloride was prepared according to the formula of Preparation Example 6a. This preparation was shown to have high storage stability (change in appearance, amount of remaining active ingredient) in the preceding experiment. During the preparation process, the granules were dried for 15 minutes. The moisture content of the finished granule preparation was 1.08%. The granules were further dried to the six different moisture contents shown in the table below and the resulting granule preparations with different moisture contents were packed in aluminum packages, 5g each. The packages were stored at 40°C for 6 months and the stability of the preparation over time was examined. The moisture content was determined based on the weight loss on drying and was determined following the general protocol for testing weight loss on drying of Japanese Pharmacopoeia.
The results are shown in Table 17 below.

**(Table 17)**

| % initial moisture content | Initial appearance | After 6-month storage (40°C) | |
|---|---|---|---|
| | | Appearance | % amount remained |
| 1.08 | Pale yellow | Pale yellow to pale reddish yellow | 100.4 |
| 0.86 | Pale yellow | Pale yellow | 102.5 |
| 0.66 | Pale yellow | Pale yellow | 102.2 |
| 0.44 | Pale yellow | Pale yellow | 99.0 |
| 0.20 | Pale yellow | Pale yellow | 101.6 |
| 0.16 | Pale yellow | Pale yellow | 102.3 |

The results demonstrate that the preparations are stable when the moisture content is 0.9% or less.
To ensure stability of the appearance of the preparation over time, it is important to control the moisture content of the preparation: The moisture content of the preparation should be decreased during the production.
As described, production of the granule preparation containing 10% sapropterin hydrochloride having the formula of Preparation Example 6a involves a drying process. We have conducted an experiment by carrying out the drying process under different conditions.
Specifically, the drying process is a part of the granulation process and involves drying extruded granules in a fluidized bed dryer. In this experiment, 500 g of materials were used to make a granule preparation of Preparation Example 6a containing 10% sapropterin hydrochloride. The drying process was carried out at a fixed aspiration temperature of 80°C over a time period of 15 or 30 minutes. The moisture content (as determined by weight loss on drying) of the preparation was then measured. The granule preparation was produced in two lots, each consisting of two batches. The product was evaluated by batch. The results are shown in Table 18 below.

**(Table 18)**

| | Drying time (min) | % moisture content (loss on drying) |
|---|---|---|
| Lot 1 (Batch 1) | 15 | 0.78 |
| Lot 1 (Batch 2) | 15 | 0.73 |
| Lot 2 (Batch 1) | 30 | 0.62 |
| Lot 2 (Batch 2) | 30 | 0.58 |

As shown in Table 18, the moisture content of the preparation was reduced to the value determined in Example 7 (*i.e*., 0.9% or less) by drying the granules for 15 minutes or longer.
Thus, it has been demonstrated that the stability of sapropterin hydrochloride and the pharmaceutical preparation of the present invention can be ensured by drying the formed granules in a fluidized bed dryer for 15 minutes or longer to adjust the moisture content of the preparation (loss on drying). The drying process may be carried out at any temperature that does not damage sapropterin hydrochloride, the constituents of the preparation, and the pharmaceutical preparation of the present invention. If desired, the granules may be dried under reduced pressure.

However, decreasing the moisture content to the degree described above will become difficult when the preparation is produced in larger scale. The moisture content can increase during the packaging process after drying or in the preparation after shipping. It is thus important to minimize the increase in the moisture content of the preparation after production.
One approach we had conceived of was to contain the preparation in a package that blocks external moisture and with a desiccant to keep the internal moisture, if any, from affecting the preparation. We therefore conducted an experiment to see if it would be possible to prevent the color change of the preparation over time by designing a package that keeps moisture out and by packaging the preparation with a desiccant to reduce the moisture present in the package.

The granules of Preparation Example 6a containing 10% sapropterin hydrochloride were packaged in three different forms, as follows: The preparation was separately packed in 21 regular cellophane-polyethylene laminate pouches, 1 g each. The pouches were then packaged in a sealed aluminum bag with 3 g of silica gel desiccant. The same preparation was separately packed in 21 high moisture-proof cellophane-polyethylene laminate pouches, 1g each, and the pouches were packaged in a sealed aluminum bag with 3 g of silica gel desiccant. The same preparation was also separately packed in pouches made of aluminum sheet containing a desiccant, 1 g each. The three types of packages were stored at 25°C/60% RH and 40°C/75% RH and were subsequently evaluated for the stability over time based on changes in the appearance, the amount of the remaining active ingredient, the moisture content (weight loss on drying) and the disintegration time. The regular cellophane-polyethylene laminate pouch used was made of regular cellophane #300/40µ polyethylene while the high moisture-proof cellophane-polyethylene laminate pouch was made of high moisture-proof cellophane #300/40µ polyethylene. The results are shown in Tables 19, 20 and 21 below.

**(Table 19)**

| Regular cellophane-polyethylene laminate pouch/aluminum bag (with desiccant) | | | | |
|---|---|---|---|---|
| Evaluation criteria | Appearance | % amount of remaining active ingredient | Loss on drying (%) | Disintegration time (min) |
| Initial | Pale yellow | 100 | 0.40 - 0.43 | Within 1 min |
| 40°C/75% RH 6 months | No change | 98.6 - 98.9 | 0.31 - 0.34 | Within 1 min |

**(Table 20)**

| High moisture-proof cellophane-polyethylene laminate pouch/aluminum bag (with desiccant) | | | | |
|---|---|---|---|---|
| Evaluation criteria | Appearance | % amount of remaining active ingredient | Loss on drying (%) | Disintegration time (min) |
| Initial | Pale yellow | 100 | 0.36 - 0.41 | Within 1 min |
| 40°C/75% RH 6 months | No change | 99.8 - 100.1 | 0.32 - 0.36 | Within 1 min |

**(Table 21)**

| Aluminum bag with desiccant | | | | |
|---|---|---|---|---|
| Evaluation criteria | Appearance | % amount of remaining active ingredient | Loss on drying (%) | Disintegration time (min) |
| Initial | Pale yellow | 100 | 0.37 - 0.42 | Within 1 min |
| 40°C/75% RH 6 months | No change | 97.5 - 98.4 | 0.44 - 0.48 | Within 1 min |
| 25°C/60% RH 39 months | No change | 97.8 - 98.6 | 0.51 - 0.53 | Within 1 min |

These results indicate that each of the three forms of packaging was effective in maintaining sufficient stability of the preparation. We further evaluated the regular cellophane-polyethylene laminate pouches and the high moisture-proof cellophane-polyethylene laminate pouches by storing them at 25° C/60% RH and 25°C/75% RH for 2 weeks. The preparation packed in the regular cellophane-polyethylene laminate pouches turned dark color and damp after 1 week at 60% RH, whereas the preparation packed in the high moisture-proof cellophane-polyethylene laminate pouches did not change color or appearance after 2 weeks at 75% RH. The weight loss on drying for the granules was 0.83%. These observations suggest that the stability of sapropterin hydrochloride can be maintained by using high water-proof packaging material. The high water-proof packaging material preferably has a moisture permeability of less than 30 g/m²/day.

Thus, it has been demonstrated that the decomposition of sapropterin hydrochloride caused by moisture can be prevented by keeping the moisture content of the preparation at 0.9% or lower during the production and containing the preparation in a package that blocks external moisture. A desiccant may be packed with the preparation to ensure the stability should moisture enter the package.

Other exemplary preparations provided in accordance with the present invention are shown in Table 22 below (amount of components given in mg). When the amount of sapropterin hydrochloride is small, the components of the preparation are preferably dissolved in water for mixing since otherwise the sapropterin hydrochloride is non-uniformly dispersed in the preparation. However, the components in the form of powder may be mixed with each other. Other pharmaceutically acceptable ingredients may be used without particular limitation, including binders, excipients, disintegrating agents and flavor. These ingredients may be used in any amounts that do not affect the granulation process. The preparation of the present invention can be produced by any common technique. Two exemplary production processes are described in the following.

### Exemplary process 1 for producing a preparation according to the formula of Preparation Example 6a

Sapropterin hydrochloride, D-mannitol, low-substituted hydroxypropylcellulose (L-HPC) and dibasic calcium phosphate dihydrate were mixed together in a high-speed mixer. To this mixture, a separately prepared binder solution and a predetermined amount of water were added and the mixture was mixed/kneaded together in a high-speed mixer. The binder solution was prepared by dissolving povidone, ascorbic acid, and L-cysteine hydrochloride in purified water to form an aqueous solution and dispersing riboflavin in the aqueous solution. Using an extrusion granulator, the kneaded product was extruded into granules, which in turn were dried in a fluidized bed dryer and sized on a vibration sieve to obtain a desired granule preparation.

### Exemplary process 2 for producing a preparation according to the formula of Preparation Example 13

D-mannitol and low-substituted hydroxypropylcellulose were mixed together in a high-speed mixer. To this mixture, a separately prepared sapropterin hydrochloride solution and a predetermined amount of water were added and the mixture was mixed/kneaded. The sapropterin hydrochloride solution was prepared by dissolving sapropterin hydrochloride, povidone, ascorbic acid and L-cysteine hydrochloride in purified water to form an aqueous solution and dispersing riboflavin in the aqueous solution. Using an extrusion granulator, the kneaded product was extruded into granules, which in turn were dried and flavored in a fluidized bed dryer and were sorted on a vibration sieve to obtain a desired granule preparation.

**(Table 22)**

| Preparation Examples | 12 | 13 | 14 | 15 | 16 |
|---|---|---|---|---|---|
| Sapropterin hydrochloride | 100.0 | 50.0 | 50.0 | 200.0 | 200.0 |
| D-mannitol | q.s. | q.s. | q.s. | q.s. | q.s. |
| L-HPC | 50.0 | 50.0 | 50.0 | 50.0 | 50.0 |
| Flavors | | | | | |
| Dibasic calcium phosphate dihydrate | 30.0 | 15.0 | | 60.0 | |
| Magnesium aluminometasilicate | | | 3.0 | | 8.0 |
| Povidone | 12.0 | 12.0 | 12.0 | 12.0 | 12.0 |
| Ascorbic acid | - | 5.0 | - | - | 5.0 |
| L-cysteine hydrochloride | - | 2.5 | - | - | 2.5 |
| Coloring agent Riboflavin | Trace Amount | Trace amount | Trace amount | Trace amount | Trace amount |
| Total | 1,000 | 1,000 | 1,000 | 1,000 | 1,000 |

As set forth, the present invention provides an effective sapropterin hydrochloride preparation that is highly stable during storage and can administer sapropterin hydrochloride, an effective therapeutic agent for the treatment of tetrahydrobiopterin-responsive hyperphenylalaninemia, to patients of a wide range of ages, ranging from infants to adults, in a single preparation.
The preparation of the present invention is particularly advantageous in that the flavoring agent used in the preparation serves to reduce the sour taste and acridity caused by the presence of sapropterin hydrochloride. The use of such flavoring agent has never been contemplated in the art. It also contains a stable coloring agent to oxidation serves to ensure stability of the preparation by concealing the color change of the preparation.

The present invention also ensures stability of the active ingredient sodium bicarbonate by controlling the moisture content of the preparation. Specifically, the moisture content is controlled during the production of the preparation or by means of special packaging.

While the present invention has been described in detail with reference to various examples and test examples, the exemplary preparations, ingredients and their amounts described herein are presented by way of example only and do not limit the invention in any way. It should be appreciated that other preparations and ingredients of equivalent functions, as well as changes in the amounts of such ingredients, are also encompassed by the scope of the invention as long as such equivalents and changes are intended for use in the treatment of tetrahydrobiopterin-responsive hyperphenylalaninemia.

### INDUSTRIAL APPLICABILITY

The present invention provides an effective sapropterin hydrochloride preparation that is highly stable during storage and can administer sapropterin hydrochloride, an effective therapeutic agent for the treatment of tetrahydrobiopterin-responsive hyperphenylalaninemia, to patients of a wide range of ages, ranging from infants to adults, in a single preparation. The sapropterin hydrochloride preparation provided by the present invention is the only effective cure for BH4-responsive hyperphenylalaninemia and BH4-responsive PAH deficiency and, thus, is of significant medical importance.

## Claims

1. A pharmaceutical preparation for the treatment of BH4-responsive hyperphenylalaninemia provided in the form of granules, fine granules or dry syrup, comprising:
2.5 to 20 wt% of sapropterin hydrochloride as an active ingredient; and
magnesium aluminometasilicate or dibasic calcium phosphate dihydrate as a flavoring agent;
wherein the preparation is suitable for use in patients of all ages, ranging from infants to adults.

2. The pharmaceutical preparation according to claim 1, further containing a coloring agent which is stable against acid and oxidation.

3. The pharmaceutical preparation according to claim 1 or 2, wherein the moisture content of the preparation is 0.9% or less.

4. The pharmaceutical preparation according to any of claims 1 to 3, further containing ascorbic acid or L-cysteine hydrochloride as a stabilizer.

5. The pharmaceutical preparation according to any of claims 1 to 4, containing 5 to 10 wt% of sapropterin hydrochloride as an active ingredient.

6. The pharmaceutical preparation according to any of claims 1 to 5, wherein during the preparation process, the flavoring agent is mixed and kneaded with sapropterin hydrochloride in the presence of water.

7. The pharmaceutical preparation according to claim 2, wherein the coloring agent is riboflavin.

8. The pharmaceutical preparation according to any of claims 1 to 7, wherein the moisture content of the preparation (loss on drying) is controlled by drying formed granules in a fluidized bed dryer.

9. The pharmaceutical preparation according to any of claims 1 to 8, wherein the moisture content of the preparation (loss on drying) is controlled by drying formed granules in a fluidized bed dryer for 15 minutes or more.

10. The pharmaceutical preparation according to any of claims 1 to 9, wherein the granule, fine granule, or dry syrup is contained in a package that blocks external moisture.

11. The pharmaceutical preparation according to any of claims 1 to 10, wherein the granule, fine granule, or dry syrup is contained in a package that blocks external moisture using a moisture-proof packaging material.

12. The pharmaceutical preparation according to claim 11, wherein the moisture-proof packaging material has a moisture permeability of less than 30 g/m²/day.

13. The pharmaceutical preparation according to claim 1, further containing riboflavin as a coloring agent, wherein the preparation has a moisture content of 0.9% or less and the preparation comprises 5 to 10 wt% of sapropterin hydrochloride.

## Patentansprüche

1. Pharmazeutisches Präparat für die Behandlung von BH4-responsiver Hyperphenylalaninämie, das in der Form von Granulat, feinem Granulat oder trockenem Sirup bereitgestellt wird, umfassend:
2,5 bis 20 Gew.-% Sapropterin-Hydrochlorid als aktives Ingrediens und
Magnesiumaluminometasilicat oder dibasisches Calciumphosphat-Dihydrat als Aromatisierungsmittel;
wobei das Präparat zur Verwendung bei Patienten jeden Alters, von Säuglingen bis Erwachsenen, geeignet ist.

2. Pharmazeutisches Präparat gemäß Anspruch 1, das außerdem ein Färbemittel enthält, das gegen Säure und Oxidation stabil ist.

3. Pharmazeutisches Präparat gemäß Anspruch 1 oder 2, wobei der Feuchtigkeitsgehalt des Präparates 0,9% oder weniger ist.

4. Pharmazeutisches Präparat gemäß einem der Ansprüche 1 bis 3, das außerdem Ascorbinsäure oder L-Cystein-Hydrochlorid als Stabilisator enthält.

5. Pharmazeutisches Präparat gemäß einem der Ansprüche 1 bis 4, das 5 bis 10 Gew.-% Sapropterin-Hydrochlorid als aktives Ingrediens enthält.

6. Pharmazeutisches Präparat gemäß einem der Ansprüche 1 bis 5, wobei während des Herstellungsverfahrens das Aromatisierungsmittel mit Sapropterin-Hydrochlorid in Gegenwart von Wasser vermischt und geknetet wird.

7. Pharmazeutisches Präparat gemäß Anspruch 2, wobei das Färbemittel Riboflavin ist.

8. Pharmazeutisches Präparat gemäß einem der Ansprüche 1 bis 7, wobei der Feuchtigkeitsgehalt des Präparates (Verlust bei Trocknung) durch Trocknung von gebildetem Granulat in einem Wirbelbetttrockner reguliert wird.

9. Pharmazeutisches Präparat gemäß einem der Ansprüche 1 bis 8, wobei der Feuchtigkeitsgehalt des Präparates (Verlust bei Trocknung) durch Trocknung von gebildetem Granulat in einem Wirbelbetttrockner für 15 Minuten oder mehr reguliert wird.

10. Pharmazeutisches Präparat gemäß einem der Ansprüche 1 bis 9, wobei das Granulat, das feine Granulat oder der trockene Sirup in einer Packung enthalten ist, die äußere Feuchtigkeit blockiert.

11. Pharmazeutisches Präparat gemäß einem der Ansprüche 1 bis 10, wobei das Granulat, das feine Granulat oder der trockene Sirup in einer Packung enthalten ist, die äußere Feuchtigkeit blockiert, indem ein feuchtigkeitsbeständiges Verpackungsmaterial verwendet wird.

12. Pharmazeutisches Präparat gemäß Anspruch 11, wobei das feuchtigkeitsbeständige Verpackungsmaterial eine Feuchtigkeitspermeabilität von weniger als 30 g/m²/Tag hat.

13. Pharmazeutisches Präparat gemäß Anspruch 1, das außerdem Riboflavin als Färbemittel enthält, wobei das Präparat einen Feuchtigkeitsgehalt von 0,9% oder weniger hat und das Präparat 5 bis 10 Gew.-% Sapropterin-Hydrochlorid umfasst.

## Revendications

1. Préparation pharmaceutique pour le traitement de l'hyperphénylalaninémie répondant à la BH4, fournie sous la forme de granulés, de granulés fins ou de sirop sec, comprenant
2,5 à 20 % en poids de chlorhydrate de saproptérine comme ingrédient actif ; et
de l'aluminométasilicate de magnésium ou du phosphate de calcium dibasique dihydraté comme agent aromatisant ;
dans laquelle la préparation est appropriée pour l'utilisation chez les patients de tous âges, allant du nourrisson à l'adulte.

2. Préparation pharmaceutique selon la revendication 1, contenant en outre un agent colorant qui est stable à l'acide et à l'oxydation.

3. Préparation pharmaceutique selon la revendication 1 ou 2, dans laquelle la teneur en humidité de la préparation est de 0,9 % ou moins.

4. Préparation pharmaceutique selon l'une quelconque des revendications 1 à 3, contenant en outre de l'acide ascorbique ou du chlorhydrate de L-cystéine comme stabilisant.

5. Préparation pharmaceutique selon l'une quelconque des revendications 1 à 4, contenant 5 à 10 % en poids de chlorhydrate de saproptérine comme ingrédient actif.

6. Préparation pharmaceutique selon l'une quelconque des revendications 1 à 5, dans laquelle, pendant le processus de préparation, l'agent aromatisant est mélangé et pétri avec du chlorhydrate de saproptérine en présence d'eau.

7. Préparation pharmaceutique selon la revendication 2, dans laquelle l'agent colorant est la riboflavine.

8. Préparation pharmaceutique selon l'une quelconque des revendications 1 à 7, dans laquelle la teneur en humidité de la préparation (perte au séchage) est contrôlée par le séchage des granulés formés dans un séchoir à lit fluidisé.

9. Préparation pharmaceutique selon l'une quelconque des revendications 1 à 8, dans laquelle la teneur en humidité de la préparation (perte au séchage) est contrôlée par le séchage des granulés formés dans un séchoir à lit fluidisé pendant 15 minutes ou plus.

10. Préparation pharmaceutique selon l'une quelconque des revendications 1 à 9, dans laquelle les granulés, les granulés fins ou le sirop sec sont contenus dans un emballage qui bloque l'humidité externe.

11. Préparation pharmaceutique selon l'une quelconque des revendications 1 à 10, dans laquelle les granulés, les granulés fins ou le sirop sec sont contenus dans un emballage qui bloque l'humidité externe à l'aide d'un matériau d'emballage résistant à l'humidité.

12. Préparation pharmaceutique selon la revendication 11, dans laquelle le matériau d'emballage résistant à l'humidité a une perméabilité à l'humidité inférieure à 30 g/m²/jour.

13. Préparation pharmaceutique selon la revendication 1, contenant en outre de la riboflavine comme agent colorant, dans laquelle la préparation a une teneur en humidité de 0,9 % ou moins et la préparation comprend 5 à 10 % en poids de chlorhydrate de saproptérine.
